# EUROPEAN PATENT APPLICATION

(11) **EP 3 244 603 A1**
(43) Date of publication of application: **15.11.2017**
(21) Application number: 15876948.9
(22) Date of filing: 29.10.2015
(51) Int. Cl.: H04N 5/225, A61B 1/04, H01L 23/12, H01L 23/32, H05K 9/00

(54) **IMAGING UNIT, IMAGING MODULE AND ENDOSCOPIC SYSTEM**

(30) Priority: 05.01.2015 JP 2015000496
(71) Applicant: Olympus Corporation, Hachioji-shi Tokyo 192-8507 (JP)
(72) Inventor: ISHIKAWA, Shinya, Tokyo 192-8507 (JP); WATAYA, Yuichi, Tokyo 192-8507 (JP); MURAMATSU, Akira, Tokyo 192-8507 (JP)
(74) Representative: Gunzelmann, Rainer
(86) International application number: PCT/JP2015/080527
(87) International publication number: WO 2016/111075

(57) **Abstract**

Provided are an imaging unit, an imaging module, and an endoscope system that make it possible to achieve a reduced diameter of a distal end of an insertion section and to obtain a high-quality image. An imaging unit 10 according to the present invention has a semiconductor package 20 which includes an image sensor and a connection electrode 21 formed on a face f2, a first multi-layer substrate 30 which includes connection electrodes 31, 33 formed on faces f3 and f4 and is connected to the semiconductor package 20 through the connection electrode 31, a second multi-layer substrate 40 which is connected to the first multi-layer substrate 30 with a layer direction of the second multi-layer substrate 40 perpendicular to a layer direction of the first multi-layer substrate 30, an electronic component 51 which is mounted inside the first multi-layer substrate 30, and a cable 60 which is connected to the second multi-layer substrate 40. The second multi-layer substrate 40 is connected to the first multi-layer substrate 30 to form a T shape, and the first multi-layer substrate 30 and the second multi-layer substrate 40 lie within a projected plane in an optical axis direction of the semiconductor package 20.

## Description

### Field

The present invention relates to an imaging unit disposed on a distal end of an insertion section of an endoscope that is configured to be inserted into a subject to image an inside of the subject. The present invention also relates to an imaging module and an endoscope system. Background

Conventionally, in the medical field and the industrial field, endoscope apparatuses have been widely used for various examinations. Among these endoscope apparatuses, a medical endoscope apparatus is capable of acquiring an in-vivo image inside the body cavity without making an incision on a subject such as a patient by inserting an elongated and flexible insertion section including an image sensor disposed on the distal end thereof into the body cavity of the subject and further capable of performing a therapeutic treatment by allowing a treatment tool to project from the distal end of the insertion section as needed, and thus widely used.

An imaging unit which includes an image sensor and a circuit board on which electronic components such as a capacitor and an IC chip which constitute a drive circuit for the image sensor are mounted is inserted in the distal end of the insertion section of such an endoscope apparatus, and a signal cable is soldered to the circuit board of the imaging unit.

In recent years, there has been proposed an imaging unit that achieves a three-dimensional structure of a circuit board connected to an image sensor for the purpose of simplifying a signal line connecting operation in a cable, improving the reliability of a connected part, or reducing the size.

### Citation List

### Patent Literature

Patent Literature 1: JP 2005-278760 A
Patent Literature 2: JP 2006-223624 A
Patent Literature 3: JP 2000-199863 A
Patent Literature 4: JP 2013-197501 A
Patent Literature 5: JP 2014-110847 A

### Summary

### Technical Problem

However, in Patent Literatures 1 to 5, since an electronic component such as a bypass capacitor is mounted at a position away from an image sensor, the impedance of wiring is increased, which causes low image quality due to generation of noise.

The present invention has been made in view of the foregoing, and an object of the invention is to provide an imaging unit, an imaging module and an endoscope system that make it possible to achieve a reduced diameter of a distal end of an insertion section and to obtain a high-quality image.

### Solution to Problem

In order to solve the above described problem and achieve the object, an imaging unit includes: a semiconductor package having an image sensor and having a connection electrode on a back face of the semiconductor package; a first multi-layer substrate having a plurality of layered substrates and having connection electrodes respectively on a front face and on a back face of the first multi-layer substrate, the connection electrode on the front face being configured to be electrically and mechanically connected to the connection electrode of the semiconductor package; a second multi-layer substrate having a plurality of layered substrates, the second multi-layer substrate being configured to be electrically and mechanically connected to the back face of the first multi-layer substrate such that a layer direction of the second multi-layer substrate is perpendicular to a layer direction of the first multi-layer substrate; an electronic component mounted inside the first multi-layer substrate; and a plurality of cables configured to be electrically and mechanically connected to the second multi-layer substrate. The second multi-layer substrate is connected to the back face of the first multi-layer substrate to form a T shape, and the first multi-layer substrate and the second multi-layer substrate lie within a projected plane in an optical axis direction of the semiconductor package.

In the imaging unit according to the above-described invention, a plurality of electronic components is mounted inside the first multi-layer substrate. In layers of the first multi-layer substrate in which the plurality of electronic components is mounted, an arrangement region for a plurality of vias connecting the layered substrates and a mounting region for the plurality of electronic components are sectioned so as to be adjacent to each other.

In the imaging unit according to the above-described invention, a plurality of electronic components is mounted inside the first multi-layer substrate. In layers of the first multi-layer substrate in which the plurality of electronic components is mounted, the plurality of electronic components is mounted in a central part of the first multi-layer substrate, and a plurality of vias connecting the layered substrates is arranged along an outer periphery of the first multi-layer substrate.

In the imaging unit according to the above-described invention, at least one of the plurality of cables is connected to the via arranged along the outer periphery of the first multi-layer substrate.

In the imaging unit according to the above-described invention, the plurality of cables connected to the second multi-layer substrate is arranged at different positions along the optical axis direction. The cable connected to the second multi-layer substrate at a position closer to the first multi-layer substrate is connected to the electronic component mounted inside the first multi-layer substrate.

In the imaging unit according to the above-described invention, the first multi-layer substrate has, on the back face thereof, a recess into which an end part of the second multi-layer substrate is inserted, and the electronic component mounted inside the first multi-layer substrate is arranged at a position different from a position of the recess.

In the imaging unit according to the above-described invention, the second multi-layer substrate is connected to the first multi-layer substrate at a position shifted from a center of the first multi-layer substrate so as to divide the projected plane in the optical axis direction of the image sensor into a wide projected plane and a narrow projected plane by the second multi-layer substrate. The cable having a larger outer diameter is connected to a face corresponding to the wide projected plane. The cable having a smaller outer diameter is connected to a face corresponding to the narrow projected plane.

In the imaging unit according to the above-described invention, the second multi-layer substrate is connected to the first multi-layer substrate with the second multi-layer substrate inclined with respect to a horizontal direction.

In the imaging unit according to the above-described invention, the first multi-layer substrate, the second multi-layer substrate, and the plurality of cables lie within the projected plane in the optical axis direction of the image sensor.

An imaging module according to the invention includes: a semiconductor package having an image sensor and having a connection electrode on a back face of the semiconductor package; a first multi-layer substrate having a plurality of layered substrates and having connection electrodes respectively on a front face and on a back face of the first multi-layer substrate, the connection electrode on the front face being configured to be electrically and mechanically connected to the connection electrode of the semiconductor package; a second multi-layer substrate having a plurality of layered substrates, the second multi-layer substrate being configured to be electrically and mechanically connected to the back face of the first multi-layer substrate such that a layer direction of the second multi-layer substrate is perpendicular to a layer direction of the first multi-layer substrate; and an electronic component mounted inside the first multi-layer substrate. The second multi-layer substrate is connected to the back face of the first multi-layer substrate to form a T shape, and the first multi-layer substrate and the second multi-layer substrate lie within a projected plane in an optical axis direction of the semiconductor package.

An endoscope system according to the invention includes an insertion section having the imaging unit according to the above-described invention disposed on a distal end of the insertion section.

### Advantageous Effects of Invention

According to the present invention, an electronic component is mounted inside a first multi-layer substrate adjacent to a semiconductor package. With this structure, it is possible to achieve a reduced diameter and to prevent low image quality caused by generation of noise.

### Brief Description of Drawings

FIG. 1 is a diagram schematically illustrating the entire configuration of an endoscope system according to a first embodiment of the present invention.
FIG. 2 is a sectional view of an imaging unit which is disposed on the distal end of an endoscope illustrated in FIG. 1.
FIG. 3 is an A-A sectional view of the imaging unit illustrated in FIG. 2.
FIG. 4 is a sectional view of an imaging unit according to a first modification of the first embodiment of the present invention.
FIG. 5 is a side view of an imaging unit according to a second modification of the first embodiment of the present invention.
FIG. 6 is a diagram describing an arrangement configuration of a second multi-layer substrate and cables of the imaging unit illustrated in FIG. 5.
FIG. 7 is a sectional view of an imaging unit according to a second embodiment of the present invention.
FIG. 8 is a B-B sectional view of the imaging unit illustrated in FIG. 7.
FIG. 9 is a sectional view of an imaging unit according to a first modification of the second embodiment of the present invention.
FIG. 10 is a sectional view of an imaging unit according to a third embodiment of the present invention.
FIG. 11 is a plan view of a first multi-layer substrate used in the imaging unit of FIG. 10.

### Description of Embodiments

An endoscope system having an imaging unit will be described below as modes for carrying out the present invention (hereinafter, referred to as "embodiment(s)"). The invention is not limited to the embodiments. The same reference signs are used to designate the same elements throughout the drawings. It is to be noted that the drawings are schematic drawings, and the relationship between the thickness and the width in each member and the ratio of each member are different from the actual relationship and ratio. The dimension and the ratio may partially differ from each other also between the drawings.

### (First Embodiment)

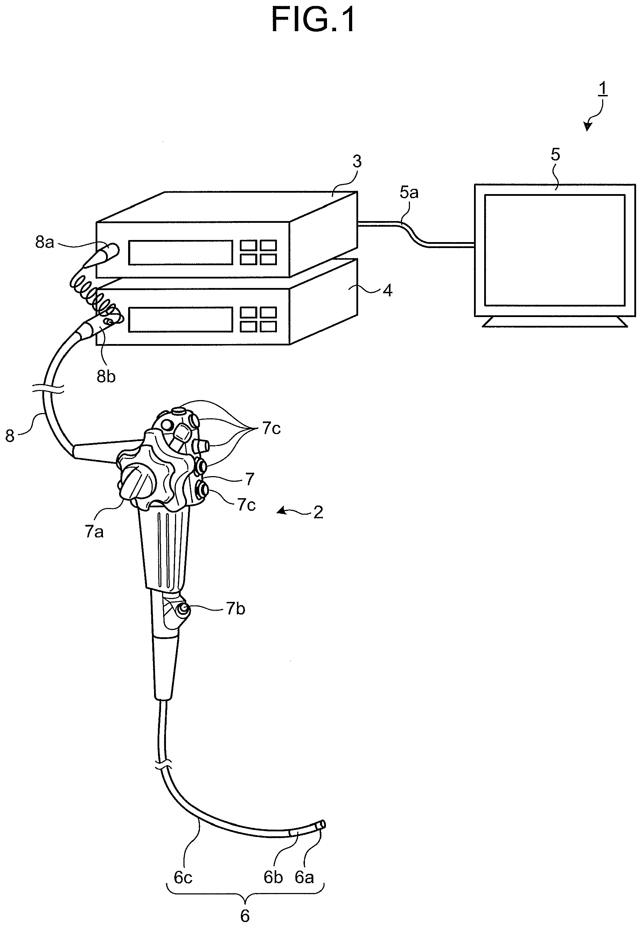

FIG. 1 is a diagram schematically illustrating the entire configuration of an endoscope system according to a first embodiment of the present invention. As illustrated in FIG. 1, the endoscope system 1 according to the first embodiment has an endoscope 2 which is configured to be introduced into a subject and to image the inside of the subject to generate an image signal of the subject, an information processing device 3 which is configured to perform predetermined image processing on the image signal captured by the endoscope 2 and controls each unit of the endoscope system 1, a light source device 4 which is configured to produce illumination light of the endoscope 2, and a display device 5 which is configured to display the image signal after the image processing by the information processing device 3.

The endoscope 2 includes an insertion section 6 which is configured to be inserted into a subject, an operating unit 7 which is located at the proximal end side of the insertion section 6 and is configured to be grasped by an operator, and a flexible universal cord 8 which extends from the operating unit 7.

The insertion section 6 is achieved using, for example, an illumination fiber (light guide cable), an electric cable, and an optical fiber. The insertion section 6 includes a distal end part 6a which has a built-in imaging unit (described below), a bendable part 6b which includes a plurality of bending pieces so as to be freely bendable, and a flexible tube part 6c which is disposed at the proximal end side of the bendable part 6b. The distal end part 6a is provided with an illumination unit which illuminates the inside of a subject through an illumination lens, an observation unit which images the inside of a subject, an opening with which a treatment tool channel communicates, and an air/water feeding nozzle (not illustrated).

The operating unit 7 includes a bending knob 7a which bends the bendable part 6b in up-down and right-left directions, a treatment tool insertion part 7b from which a treatment tool such as a biopsy forceps or a laser scalpel is inserted into the body cavity of a subject, and a plurality of switches 7c for operating peripheral devices including the information processing device 3, the light source device 4, an air feeding device, a water feeding device, and a gas feeding device. A treatment tool inserted from the treatment tool insertion part 7b passes through the treatment tool channel formed inside thereof and is exposed from the opening formed on the distal end of the insertion section 6.

The universal cord 8 is configured using, for example, an illumination fiber, an electric cable, and an optical fiber. The universal cord 8 is branched at the proximal end thereof, and includes a connector 8a on one end of the branched part and a connector 8b on the other proximal end thereof. The connector 8a is freely attachable to and detachable from a connector of the information processing device 3. The connector 8b is freely attachable to and detachable from the light source device 4. The universal cord 8 transmits illumination light emitted from the light source device 4 to the distal end part 6a through the connector 8b and the illumination fiber. Further, the universal cord 8 transmits an image signal captured by the imaging unit (described below) to the information processing device 3 through the cable and the connector 8a.

The information processing device 3 performs predetermined image processing on an image signal output from the connector 8a and controls the entire endoscope system 1.

The light source device 4 is configured using, for example, a light source which emits light and a condenser lens. The light source device 4 emits light from the light source and supplies the emitted light to the endoscope 2 which is connected through the connector 8b and the illumination fiber of the universal cord 8 as illumination light to the inside of a subject under the control of the information processing device 3.

The display device 5 is configured using, for example, a display using a liquid crystal or an organic electro luminescence (EL). The display device 5 displays various kinds of information including an image on which predetermined image processing has been performed by the information processing device 3 through a video cable 5a. Accordingly, an operator can perform observation and property determination for a desired position inside a subject by operating the endoscope 2 while checking an image (in-vivo image) displayed on the display device 5.

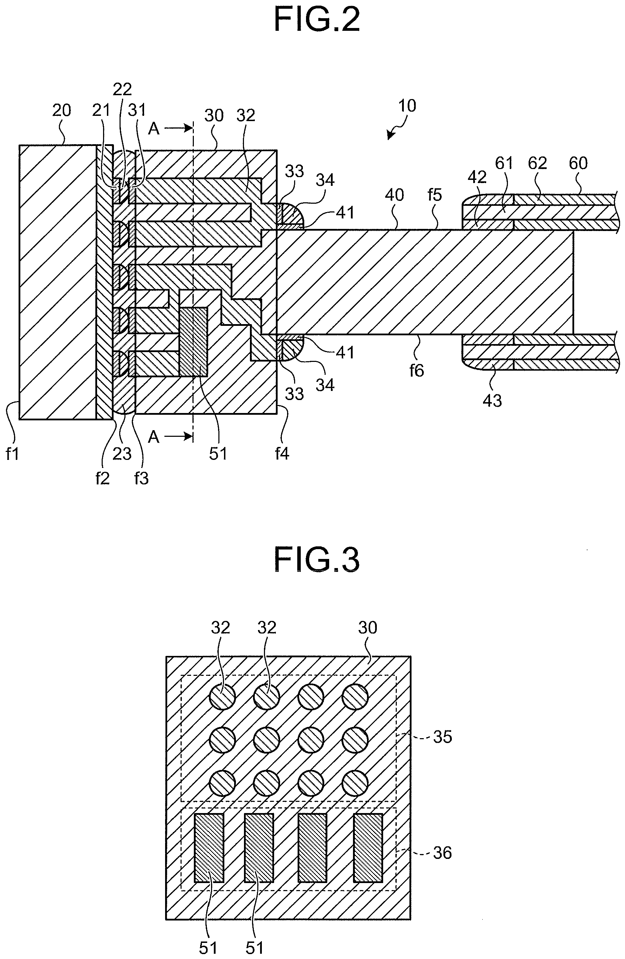

Next, the imaging unit used in the endoscope system 1 will be described in detail. FIG. 2 is a sectional view of the imaging unit which is disposed on the distal end of the endoscope illustrated in FIG. 1. FIG. 3 is an A-A sectional view of the imaging unit illustrated in FIG. 2.

An imaging unit 10 has a semiconductor package 20 which includes an image sensor and a connection electrode 21 formed on the back face thereof, a first multi-layer substrate 30 which has a plate shape and includes a connection electrode 31 formed on the front face thereof and a connection electrode 33 formed on the back face thereof, the connection electrode 31 on the front face being electrically and mechanically connected to the connection electrode 21 of the semiconductor package 20, a second multi-layer substrate 40 which has a plate shape and is electrically and mechanically connected to the back face of the first multi-layer substrate 30 with a layer direction of the second multi-layer substrate 40 perpendicular to a layer direction of the first multi-layer substrate 30, an electronic component 51 mounted inside the first multi-layer substrate 30, and a plurality of cables 60 configured to be electrically and mechanically connected to the second multi-layer substrate 40.

The image sensor of the semiconductor package 20 includes, for example, a CMOS, and a light receiver which receives light condensed by a lens unit is disposed on a face f1 as the front face. The light receiver is connected to the connection electrode 21 formed on a face f2 as the back face. A bump 22 which includes, for example, a solder is formed on the connection electrode 21. The semiconductor package 20 is preferably a chip size package (CSP) that is formed by performing wiring, electrode forming, resin sealing, and dicing on an image sensor chip in a wafer state so that the image sensor chip finally has a size equal to the size of the semiconductor package.

The first multi-layer substrate 30 has a plate shape in which a plurality of substrates having wiring is layered (a plurality of substrates parallel to a face f3 and a face f4 is layered). For example, a ceramic substrate, a glass epoxy substrate, a glass substrate, or a silicon substrate is used as each of the layered substrates. A plurality of electronic components 51 is built inside the first multi-layer substrate 30, and a plurality of vias 32 for electrically connecting the wiring on the layered substrates is formed inside the first multi-layer substrate 30. As illustrated in FIG. 3, four electronic components 51 are built into the first multi-layer substrate 30. All the electronic components 51 may be the same kind or different kinds of electronic components, and the number of built-in electronic components 51 is not limited to four. Examples of the electronic component 51 include a capacitor, a passive component such as a resistance coil, and an active component such as a driver IC. Further, the number and the arrangement of vias 32 are not limited to those illustrated in FIG. 3.

The connection electrode 31 is formed on the face f3 of the first multi-layer substrate 30, and electrically and mechanically connected to the connection electrode 21 of the semiconductor package 20 through the bump 22. A connection part between the connection electrode 31 and the connection electrode 21 is sealed with a sealing resin 23. The connection electrode 33 is formed on the face f4 of the first multi-layer substrate 30 and connected to the connection electrode 31 through the via 32.

The electronic components 51 inside the first multi-layer substrate 30 are mounted in an electronic component arrangement region 36 in substrate layers of the first multi-layer substrate 30. The electronic component arrangement region 36 is sectioned so as to be adjacent to a via arrangement region 35 in which the vias 32 are arranged. The electronic components 51 and the vias 32 can be efficiently arranged within a limited space by sectioning the electronic component arrangement region 36 and the via arrangement region 35 so as to be adjacent to each other.

The second multi-layer substrate 40 has a plate shape in which a plurality of substrates having wiring is layered (a plurality of substrates parallel to a face f5 and a face f6 is layered) similarly to the first multi-layer substrate 30. For example, a ceramic substrate, a glass epoxy substrate, a glass substrate, or a silicon substrate is used as each of the layered substrates. The second multi-layer substrate 40 is electrically and mechanically connected to the first multi-layer substrate 30 with the layer direction of the second multi-layer substrate 40 perpendicular to the layer direction of the first multi-layer substrate 30. The first multi-layer substrate 30 and the second multi-layer substrate 40 are connected to form a T shape. The face f4 which is the back face of the first multi-layer substrate 30 is equally divided into two parts by the second multi-layer substrate 40.

A connection electrode 41 is formed on one end of the second multi-layer substrate 40 and connected to the connection electrode 33 of the first multi-layer substrate 30 with a solder 34. The connection between the first multi-layer substrate 30 and the second multi-layer substrate 40 is performed in such a manner that an adhesive is applied to a predetermined position of the first multi-layer substrate 30, the second multi-layer substrate 40 is then placed and temporarily fixed, and the connection electrode 33 and the connection electrode 41 are then connected with the solder 34.

A cable connecting electrode 42 for connecting the plurality of cables 60 is formed on the other end of the second multi-layer substrate 40. In each of the cables 60, an insulating outer sheath 62 on one end is peeled, and an exposed conductor 61 is connected to the cable connecting electrode 42 with a solder 43.

In the first embodiment, the electronic components 51 are built into the first multi-layer substrate 30 which is directly connected to the semiconductor package 20. Thus, it is possible to reduce the distance between the electronic components 51 and the image sensor inside the semiconductor package 20, that is, reduce the wiring length to reduce noise. Further, since the electronic components 51 are mounted in the first multi-layer substrate 30, wiring reduction is achieved compared to the case in which the electronic components 51 are mounted in the second multi-layer substrate 40. Accordingly, the entire length of the imaging unit can be reduced.

In the first embodiment, the first multi-layer substrate 30, the second multi-layer substrate 40, and the plurality of cables 60 lie within a projected plane in an optical axis direction of the semiconductor package 20. It is therefore possible to achieve a reduced diameter of the imaging unit 10.

### (First Modification of First Embodiment)

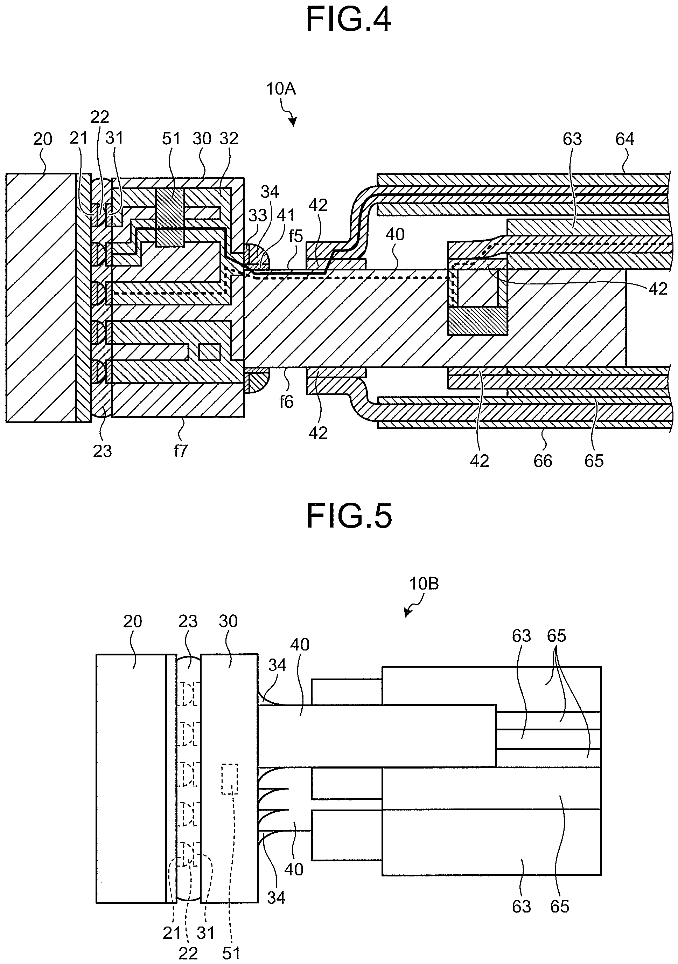

In an imaging unit according to a first modification of the first embodiment, the second multi-layer substrate 40 is connected to the back face of the first multi-layer substrate 30 at a position shifted from the center thereof. FIG. 4 is a sectional view of the imaging unit according to the first modification of the first embodiment of the present invention.

In an imaging unit 10A according to the first modification of the first embodiment, as illustrated in FIG. 4, the second multi-layer substrate 40 is connected to the back face of the first multi-layer substrate 30 at a position shifted downward (toward a face f7 of the first multi-layer substrate 30) from the center thereof. Further, cables 63, 64, 65 and 66 having different outer diameters are connected to the second multi-layer substrate 40.

When the second multi-layer substrate 40 is connected to the center of the first multi-layer substrate 30 so as to equally divide the back face of the first multi-layer substrate 30 into two parts and the cables 63 to 66 having different outer diameters are connected in any manner, the outer diameter of the imaging unit 10A may be increased. In the first modification of the first embodiment, the second multi-layer substrate 40 is connected to the back face of the first multi-layer substrate 30 at the position shifted from the center thereof so as to divide the projected plane in the optical axis direction of the image sensor into a wide projected plane and a narrow projected plane by the second multi-layer substrate 40, and the cables 63 and 64 having a large outer diameter are connected to a face (the face f5) corresponding to the wide projected plane and the cables 65 and 66 having a small outer diameter are connected to a face (the face f6) corresponding to the narrow projected plane. Accordingly, even when the cables 63 and 64 having a large outer diameter are used, it is possible to allow the first multi-layer substrate 30, the second multi-layer substrate 40, and the cables 63 and 64 to lie within the projected plane in the optical axis direction of the image sensor, thereby to achieve the reduced diameter of the imaging unit 10A.

Further, in the first modification of the first embodiment, the cables 63 to 66 are connected to the second multi-layer substrate 40 at different positions along the optical axis direction. On the face f5 of the second multi-layer substrate 40, the cable 64 is connected at a position closer to the first multi-layer substrate 30, and the cable 63 is connected at the proximal end side. On the face f6, the cable 66 is connected at a position closer to the first multi-layer substrate 30, and the cable 65 is connected at the proximal end side. The cable 64 which is connected at the position closer to the first multi-layer substrate 30 transmits an electric signal to the semiconductor package 20 through the electronic component 51 mounted inside the first multi-layer substrate 30. The electric signal is transmitted from the cable 64 to the semiconductor package 20 as indicated by a solid line in FIG. 4. Further, an electric signal is transmitted from the cable 63 to the semiconductor package 20 as indicated by a dotted line in FIG. 4. In FIG. 4, transmission paths of an electric signal between the cables 65, 66 and the semiconductor package 20 are not illustrated. Noise can be reduced by connecting the cable 64 which is connected to the electronic component 51 mounted inside the first multi-layer substrate 30 at the position closer to the first multi-layer substrate 30.

### (Second Modification of First Embodiment)

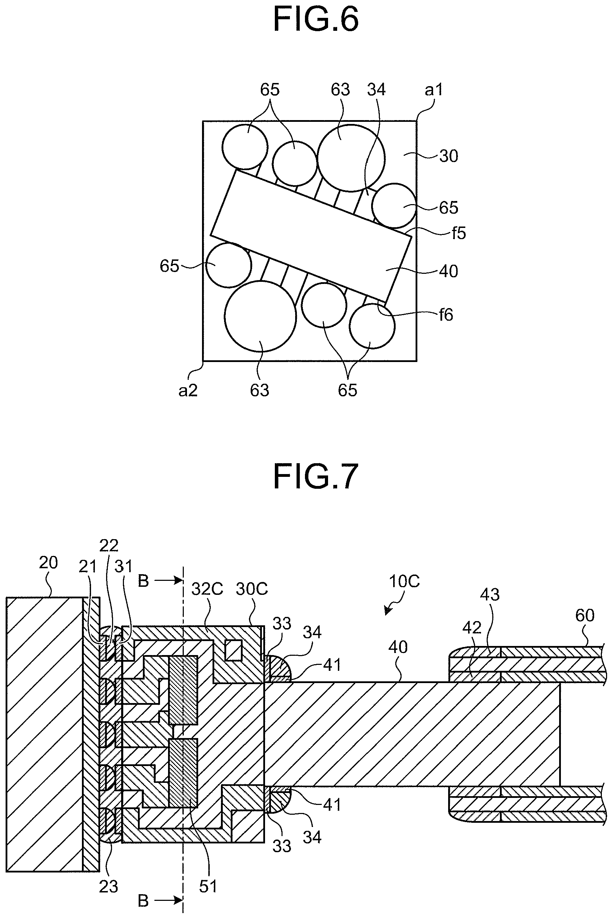

In an imaging unit according to a second modification of the first embodiment, the second multi-layer substrate 40 is connected to the first multi-layer substrate 30 with the second multi-layer substrate 40 inclined with respect to the horizontal direction. FIG. 5 is a side view of the imaging unit according to the second modification of the first embodiment of the present invention. FIG. 6 is a diagram describing an arrangement configuration between the second multi-layer substrate and cables of the imaging unit illustrated in FIG. 5.

In an imaging unit 10B according to the second modification of the first embodiment, as illustrated in FIGS. 5 and 6, the second multi-layer substrate 40 is connected to the first multi-layer substrate 30 with the second multi-layer substrate 40 inclined with respect to the horizontal direction. Cables 63 and 65 having different outer diameters are connected to the second multi-layer substrate 40. The cable 63 has a large outer diameter, and the cable 65 has a small outer diameter. In the second multi-layer substrate 40, one cable 63 and three cables 65 are connected to a face f5, and one cable 63 and three cables 65 area connected to a face f6.

When the second multi-layer substrate 40 is connected to the center of the first multi-layer substrate 30 so as to equally divide the back face of the first multi-layer substrate 30 into two parts and the cables 63 and 65 having different outer diameters are connected in any manner, the outer diameter of the imaging unit 10B may be increased. In the second modification of the first embodiment, the second multi-layer substrate 40 is connected to the first multi-layer substrate 30 with the second multi-layer substrate 40 inclined with respect to the horizontal direction, and the cables 63 having a large outer diameter are arranged near a corner a1 and a corner a2. Accordingly, even when the cables 63 having a large outer diameter are used, it is possible to allow the first multi-layer substrate 30, the second multi-layer substrate 40, and the cables 63 and 65 to lie within the projected plane in the optical axis direction of the image sensor, thereby to achieve the reduced diameter of the imaging unit 10B.

### (Second Embodiment)

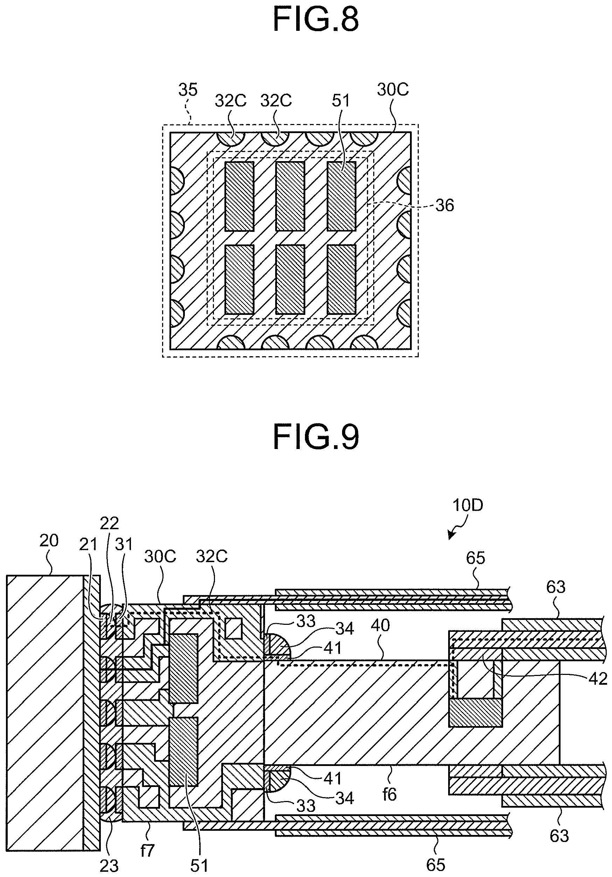

In an imaging unit according to a second embodiment, vias are arranged along the outer periphery of a first multi-layer substrate. FIG. 7 is a sectional view of the imaging unit according to the second embodiment of the present invention. FIG. 8 is a B-B sectional view of the imaging unit illustrated in FIG. 7.

In an imaging unit 10C according to the second embodiment, as illustrated in FIGS. 7 and 8, in substrate layers of a first multi-layer substrate 30C in which a plurality of electronic components 51 is mounted, the plurality of electronic components 51 is mounted in an electronic component arrangement region 36 located in the central part of the first multi-layer substrate 30C, and a plurality of vias 32C for connecting the layered substrates is arranged in a via arrangement region 35 located along the outer periphery of the first multi-layer substrate 30C.

More electronic components 51 can be mounted and more vias 32C can be arranged in the first multi-layer substrate 30C by arranging the electronic component arrangement region 36 in the central part of the first multi-layer substrate 30C and by arranging the via arrangement region 35 along the outer periphery which surrounds the electronic component arrangement region 36.

### (First Modification of Second Embodiment)

In an imaging unit according to a first modification of the second embodiment, some of the cables are connected to the vias of the first multi-layer substrate. FIG. 9 is a sectional view of the imaging unit according to the first modification of the second embodiment of the present invention.

In an imaging unit 10D according to the first modification of the second embodiment, as illustrated in FIG. 9, a cable 65 having a small outer diameter is connected to a via 32C arranged on the outer periphery of the first multi-layer substrate 30C, and a cable 63 having a large outer diameter is connected to the second multi-layer substrate 40. The cable 65 connected to the via 32C of the first multi-layer substrate 30C transmits an electric signal to the semiconductor package 20 through the electronic component 51 mounted inside the first multi-layer substrate 30C as indicated by a solid line in FIG. 9. The cable 63 connected to the second multi-layer substrate 40 transmits an electric signal to the semiconductor package 20 as indicated by a dotted line in FIG. 9. In FIG. 9, transmission paths of an electric signal between the cables 63, 65 connected to a face f7 of the first multi-layer substrate 30C and a face f6 of the second multi-layer substrate 40 and the semiconductor package 20 are not illustrated. Noise can be reduced by connecting the cable 65 which is connected to the electronic component 51 mounted inside the first multi-layer substrate 30C to the via 32C of the first multi-layer substrate 30C.

In the first modification of the second embodiment, the cable 65 having a small outer diameter is connected to the via 32C, and the first multi-layer substrate 30C, the second multi-layer substrate 40, and the cables 63 and 65 lie within the projected plane in the optical axis direction of the semiconductor package 20. Thus, it is possible to achieve the reduced diameter of the imaging unit 10D.

### (Third Embodiment)

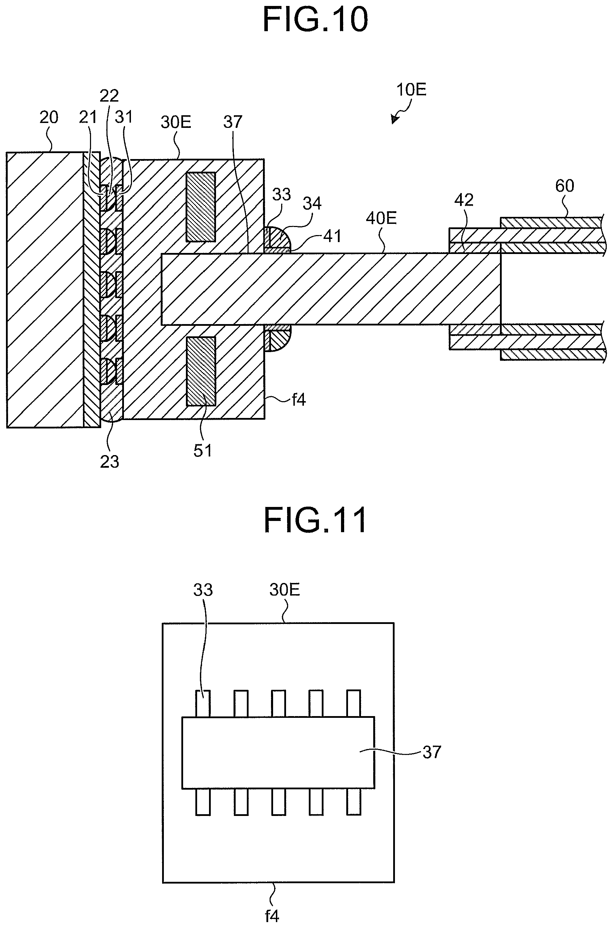

In an imaging unit according to a third embodiment, a recess is formed on the back face of a first multi-layer substrate to which a second multi-layer substrate is connected. FIG. 10 is a sectional view of the imaging unit according to the third embodiment of the present invention. FIG. 11 is a plan view of the first multi-layer substrate used in the imaging unit of FIG. 10.

In an imaging unit 10E according to the third embodiment, as illustrated in FIGS. 10 and 11, a recess 37 for inserting an end part of a second multi-layer substrate 40E is formed on a face f4 of a first multi-layer substrate 30E. The recess 37 has a size that enables the end of the second multi-layer substrate 40E to be inserted into the recess 37. The connection between the first multi-layer substrate 30E and the second multi-layer substrate 40E is performed in such a manner that the recess 37 is filled with an adhesive, the end of the second multi-layer substrate 40E is then inserted into and temporarily fixed to the recess 37, and a connection electrode 33 and a connection electrode 41 are then electrically and mechanically connected with a solder 34.

An electronic component 51 which is built into the first multi-layer substrate 30E is arranged at a position different from the position of the recess 37. In the third embodiment, the electronic component 51 is mounted on the same layer as the recess 37 in the first multi-layer substrate 30E. Thus, the electronic component 51 is mounted at a potion shifted from the position of the recess 37. When the electronic component 51 is mounted on a layer different from the layer of the recess 37, the position of the electronic component 51 and the position of the recess 37 may overlap each other. However, in order to reduce the length in the optical axis direction of the first multi-layer substrate 30E, the electronic component 51 and the recess 37 are preferably arranged at different positions on the same layer.

### Industrial Applicability

An imaging unit and an imaging module of the present invention are useful in an endoscope system that requires a high-quality image and a reduction in the diameter of the distal end thereof.

### Reference Signs List

1 ENDOSCOPE SYSTEM
2 ENDOSCOPE
3 INFORMATION PROCESSING DEVICE
4 LIGHT SOURCE DEVICE
5 DISPLAY DEVICE
6 INSERTION SECTION
6a DISTAL END PART
6b BENDABLE PART
6c FLEXIBLE TUBE PART
7 OPERATING UNIT
7a BENDING KNOB
7b TREATMENT TOOL INSERTION PART
7c SWITCH
8 UNIVERSAL CORD
8a, 8b CONNECTOR
10, 10A, 10B, 10C, 10D, 10E IMAGING UNIT
20 SEMICONDUCTOR PACKAGE
21, 31, 33, 41 CONNECTION ELECTRODE
22 BUMP
23 SEALING RESIN
30, 30C, 30E FIRST MULTI-LAYER SUBSTRATE
32, 32C VIA
34, 43 SOLDER
35 VIA ARRANGEMENT REGION
36 ELECTRONIC COMPONENT ARRANGEMENT REGION
37 RECESS
40, 40E SECOND MULTI-LAYER SUBSTRATE
42 CABLE CONNECTING ELECTRODE
51 ELECTRONIC COMPONENT
60, 63, 64, 65, 66 CABLE
61 CONDUCTOR
62 OUTER SHEATH

## Claims

1. An imaging unit comprising:
a semiconductor package having an image sensor and having a connection electrode on a back face of the semiconductor package;
a first multi-layer substrate having a plurality of layered substrates and having connection electrodes respectively on a front face and on a back face of the first multi-layer substrate, the connection electrode on the front face being configured to be electrically and mechanically connected to the connection electrode of the semiconductor package;
a second multi-layer substrate having a plurality of layered substrates, the second multi-layer substrate being configured to be electrically and mechanically connected to the back face of the first multi-layer substrate such that a layer direction of the second multi-layer substrate is perpendicular to a layer direction of the first multi-layer substrate;
an electronic component mounted inside the first multi-layer substrate; and
a plurality of cables configured to be electrically and mechanically connected to the second multi-layer substrate, wherein
the second multi-layer substrate is connected to the back face of the first multi-layer substrate to form a T shape, and
the first multi-layer substrate and the second multi-layer substrate lie within a projected plane in an optical axis direction of the semiconductor package.

2. The imaging unit according to claim 1, wherein
a plurality of electronic components is mounted inside the first multi-layer substrate, and
in layers of the first multi-layer substrate in which the plurality of electronic components is mounted, an arrangement region for a plurality of vias connecting the layered substrates and a mounting region for the plurality of electronic components are sectioned so as to be adjacent to each other.

3. The imaging unit according to claim 1, wherein
a plurality of electronic components is mounted inside the first multi-layer substrate, and
in layers of the first multi-layer substrate in which the plurality of electronic components is mounted, the plurality of electronic components is mounted in a central part of the first multi-layer substrate, and a plurality of vias connecting the layered substrates is arranged along an outer periphery of the first multi-layer substrate.

4. The imaging unit according to claim 3, wherein
at least one of the plurality of cables is connected to the via arranged along the outer periphery of the first multi-layer substrate.

5. The imaging unit according to any one of claims 1 to 3, wherein
the plurality of cables connected to the second multi-layer substrate is arranged at different positions along the optical axis direction, and
the cable connected to the second multi-layer substrate at a position closer to the first multi-layer substrate is connected to the electronic component mounted inside the first multi-layer substrate.

6. The imaging unit according to claim 1, wherein
the first multi-layer substrate has, on the back face thereof, a recess into which an end part of the second multi-layer substrate is inserted, and
the electronic component mounted inside the first multi-layer substrate is arranged at a position different from a position of the recess.

7. The imaging unit according to claim 1, wherein
the second multi-layer substrate is connected to the first multi-layer substrate at a position shifted from a center of the first multi-layer substrate so as to divide the projected plane in the optical axis direction of the image sensor into a wide projected plane and a narrow projected plane by the second multi-layer substrate, and
the cable having a larger outer diameter is connected to a face corresponding to the wide projected plane, and
the cable having a smaller outer diameter is connected to a face corresponding to the narrow projected plane.

8. The imaging unit according to claim 1, wherein
the second multi-layer substrate is connected to the first multi-layer substrate with the second multi-layer substrate inclined with respect to a horizontal direction.

9. The imaging unit according to any one of claims 1 to 8, wherein
the first multi-layer substrate, the second multi-layer substrate, and the plurality of cables lie within the projected plane in the optical axis direction of the image sensor.

10. An imaging module comprising:
a semiconductor package having an image sensor and having a connection electrode on a back face of the semiconductor package;
a first multi-layer substrate having a plurality of layered substrates and having connection electrodes respectively on a front face and on a back face of the first multi-layer substrate, the connection electrode on the front face being configured to be electrically and mechanically connected to the connection electrode of the semiconductor package;
a second multi-layer substrate having a plurality of layered substrates, the second multi-layer substrate being configured to be electrically and mechanically connected to the back face of the first multi-layer substrate such that a layer direction of the second multi-layer substrate is perpendicular to a layer direction of the first multi-layer substrate; and
an electronic component mounted inside the first multi-layer substrate, wherein
the second multi-layer substrate is connected to the back face of the first multi-layer substrate to form a T shape, and
the first multi-layer substrate and the second multi-layer substrate lie within a projected plane in an optical axis direction of the semiconductor package.

11. An endoscope system comprising an insertion section having the imaging unit according to any one of claims 1 to 9 disposed on a distal end of the insertion section.
